# EUROPÄISCHE PATENTSCHRIFT

(11) **EP 1 745 284 B1**
(45) Veröffentlichungstag und Bekanntmachung des Hinweises auf die Patenterteilung: **07.05.2008**
(21) Anmeldenummer: 05707236.5
(22) Anmeldetag: 05.02.2005
(51) Int. Cl.: G01N 33/487

(54) **ANALYSEHANDGERÄT MIT FÖRDERWEG FÜR ANALYTISCHE VERBRAUCHSMITTEL**
HANDHELD APPARATUS FOR ANALYSIS WITH A CONVEYANCE PATH FOR TEST ELEMENTS
APPAREIL D'ANALYSE A MAIN COMPORTANT UNE SECTION DE TRANSPORT POUR DES ELEMENTS D'ANALYSE

(30) Priorität: 04.03.2004 DE 102004010529
(43) Veröffentlichungstag der Anmeldung: 24.01.2007
(60) Teilanmeldung: 08005435.6
(73) Patentinhaber: Roche Diagnostics GmbH, 68305 Mannheim (DE); F.HOFFMANN-LA ROCHE AG, 4070 Basel (CH)
(72) Erfinder: SCHABBACH, Michael, 69469 Weinheim (DE)
(74) Vertreter: Jany, Peter
(86) Internationale Anmeldenummer: PCT/EP2005/001196
(87) Internationale Veröffentlichungsnummer: WO 2005/085840

(56) Entgegenhaltungen:
- EP-A- 0 640 393
- EP-A- 1 022 565
- DE-A1- 10 156 811
- US-A- 3 918 910
- US-A1- 2002 057 993

## Beschreibung

Die Erfindung betrifft ein Analysehandgerät zum Untersuchen einer Probe, insbesondere einer biologischen Flüssigkeit, auf einen medizinisch bedeutsamen Bestandteil, umfassend einen Analysesensor, dem auf einem Förderweg ein analytisches Verbrauchsmittel zuführbar ist, eine Anzeigeeinrichtung, ein Gehäuse, das eine Gehäuseöffnung für ein analytisches Verbrauchsmittel aufweist, an die der Förderweg anschließt.

Für die chemische und biochemische Analyse von festen und flüssigen Probenmaterialien haben sich in darauf spezialisierten Labors und insbesondere auch für den Einsatz außerhalb fester Labors trägergebundene Schnelltests etabliert. Solche trägergebundenen Schnelltests sind trotz der oftmals komplexen Reaktionen unter Beteiligung empfindlicher Reagenzien selbst von Laien einfach und unkompliziert durchzuführen.

Ein bekanntes Beispiel für trägergebundene Schnelltests sind Testelemente für die Bestimmung des Blutglucosegehalts bei Diabetikern. Diagnostische Testelemente, die streifenförmig ausgebildet sind, werden auch als Teststreifen bezeichnet. Bekannte Ausführungsformen sind zum Beispiel Ein- oder Mehrfelderteststreifen für die Urin analytik und diverse Indikatorpapiere. Da neben Testelementen in Streifenform auch andere Formen trägergebundener Tests existieren, spricht man allgemeiner von analytischen Verbrauchsmitteln, wozu beispielsweise auch Lanzetten oder Probenentnahmeelemente zählen.

Derartige analytische Verbrauchsmittel werden in einem tragbaren Analysehandgerät verwendet, das beispielsweise mit einem optischen Analysesensor eine Verfärbung eines Teststreifens photometrisch auswertet. Die analytischen Verbrauchsmittel können in einem Trommelmagazin gelagert, sein wie es beispielsweise in der EP 1 022 565 A2 beschrieben ist. Das dort beschriebene Trommelmagazin weist mehrere ringförmig angeordnete Kammern auf, die analytische Verbrauchsmittel enthalten können und jeweils eine Entnahmeöffnung an einer Stirnseite des Trommelmagazins aufweisen. Diese Entnahmeöffnungen sind üblicherweise mit Siegelfolie verschlossen, um die analytischen Verbrauchsmittel vor schädlichen Umwelteinflüssen wie zum Beispiel Feuchtigkeit, Licht oder Staub zu schützen.

Ein Analysehandgerät mit Stapelmagazin ist aus US 2002/0057993 bekannt.

Analysehandgeräte zur Untersuchung eines medizinisch bedeutsamen Bestandteils einer Probe, wie beispielsweise Geräte zur Bestimmung des Blutglucosegehalts, werden von einem Anwender in der Regel mehrfach täglich benutzt und ständig mitgeführt. Es besteht deshalb ein Bedürfnis, derartige Analysehandgeräte möglichst klein auszuführen und gleichzeitig möglichst leicht handhabbar zu machen.

Diese Aufgabe wird bei einem Analysehandgerät der eingangs genannten Art erfindungsgemäß dadurch gelöst, daß es eine antreibbare Förderwalze hat mit der ein in den Förderweg ragendes Verbrauchsmittel gegriffen und entlang des Förderwegs bewegt werden kann, und zwar sowohl in Entnahmerichtung als auch in entgegengesetzter Richtung.

Bei einem erfindungsgemäßen Analysehandgerät kann ein analytisches Verbrauchsmittel, auf das beispielsweise ein Tropfen Blut oder Urin aufgebracht wurde, mittels der Förderwalze wesentlich leichter mit der erforderlichen Positioniergenauigkeit dem Analysesensor im inneren des Geräts zugeführt werden, als dies bei Analysehandgeräten nach dem Stand der Technik der Fall ist.

Bevorzugt weist das Analysehandgerät einen Schalter auf, der beim Einführen eines Verbrauchsmittels in die Gehäuseöffnung betätigt wird und einen Antrieb der Förderwalze einschaltet. Ein solcher Schalter kann beispielsweise ein mechanischer Schalter sein, der durch eine kleine Rotationsbewegung der Förderwalze ausgelöst wird, die durch Druck mit einem eingeführten Verbrauchsmittel gegen die Förderwalze erzeugt wird. Der Schalter kann beispielsweise als eine Lichtschranke ausgebildet sein. Eine weitere Möglichkeit ist es, den Schalter als zwei voneinander beabstandete Kontaktfelder auszubilden, die von einem in die Förderöffnung eingeführten Verbrauchsmittel in elektrischen Kontakt gebracht werden. Durch eine geeignete Transistorschaltung kann auch eine sehr geringe Änderung des elektrischen Widerstandes zwischen den beiden Kontaktfeldern, wie sie beispielsweise durch ein eingeführtes Verbrauchsmittel aus Kunststoff oder Papier, erzeugt wird, zuverlässig erfaßt werden.

Bei einem erfindungsgemäßen Analysehandgerät muß der Anwender das Verbrauchsmittel nur mit einem Ende leicht in die Gehäuseöffnung einführen. Die Förderwalze greift dann das Verbrauchsmittel. Der Transport des Verbrauchsmittels und seine korrekte Positionierung hinsichtlich des Analysesensors erfolgt automatisch. Eine Endposition des Verbrauchsmittels kann mechanisch, elektrochemisch oder optisch mit einem Positionsschalter erfaßt werden, der daraufhin den Antrieb der Förderwalze abschaltet. Die erfindungsgemäße Maßnahme hat ferner den Vorteil, daß die Entnahmeeinrichtung kompakt ausgeführt werden kann, was insbesondere bei Verwendung eines Trommelmagazins ein im Vergleich zum Stand der Technik kleineres Analysehandgerät ermöglicht. Bekannte Analysehandgeräte mit einer Entnahmeeinrichtung für ein Trommelmagazin gemäß der EP 1 022 565 A2 benötigen eine als Stößel ausgebildete Schubstange von einer erheblichen Länge, die beim Entnehmen eines Verbrauchsmittels in eine einer Entnahmeöffnung gegenüberliegenden Einschuböffnung einer Kammer des Trommelmagazins eingeführt wird und ein in der Kammer enthaltenes Verbrauchsmittel aus der Kammer und einer Gehäuseöffnung des Geräts hinausschiebt. Damit eine Schubstange diese Funktion erfüllen kann, muß sie eine Länge aufweisen, die mindestens der Länge des Weges entspricht, auf dem ein Verbrauchsmittel beim Entnehmen transportiert wird. Um diese Schubstange beherbergen zu können, muß ein solches Analysehandgerät nach dem Stand der Technik deshalb eine beträchtliche Länge aufweisen, die mindestens der Summe aus den Längen der Schubstange und des Trommelmagazins entspricht.

Vorteilhaft kann bei einem erfindungsgemäßen Analysehandgerät die Schubstange und folglich auch das Gehäuse wesentlich verkürzt werden. Es genügt nämlich bei einem erfindungsgemäßen Analysehandgerät bereits, wenn mittels einer Schubstange ein Verbrauchsmittel so weit aus einer Kammer herausgeschoben werden kann, daß es von der Förderwalze gegriffen und in Entnahmerichtung bewegt werden kann. Bevorzugt ist die Förderwalze unmittelbar benachbart zu der Entnahmeöffnung des eingelegten Trommelmagazins angeordnet, so daß es ausreicht, wenn das Verbrauchsmittel etwa 0,5 bis 1 cm weit von der Schubstange aus seiner Kammer herausgeschoben wird. Hierfür ist bereits eine Schubstange mit einer Länge von 1 bis 2 cm ausreichend, so daß ein erfindungsgemäßes Analysehandgerät etwa 10 cm kürzer als ein Gerät nach dem Stand der Technik sein kann.

Vorteilhaft kann mittels der Förderwalze ein Verbrauchsmittel ohne Schwierigkeiten so weit aus einer Gehäuseöffnung des Gehäuses herausgeschoben werden, daß sich eine Probe, beispielsweise ein Tropfen Blut, auf das Verbrauchsmittel aufbringen läßt, ohne daß dabei eine Gefahr besteht, das Analysehandgerät mit der Probe zu verschmutzen. Diese Erleichterung bezüglich des Auftragens einer Probe auf ein analytisches Verbrauchsmittel bedeutet, daß sich ein erfindungsgemäßes Analysehandgerät wesentlich bequemer nutzen und leichter handhaben läßt. Bei bekannten Analysehandgeräten wird eine um so längere Schubstange benötigt, je weiter das Verbrauchsmittel aus der Gehäuseöffnung des Analysehandgeräts herausgeschoben werden soll. Demzufolge ist bei bekannten Analysehandgeräten eine leichtere Handhabbarkeit beim Aufbringen einer Probe auf ein Verbrauchsmittel stets mit dem Nachteil größerer Abmessungen des Analysehandgeräts verbunden.

Weitere Einzelheiten und Vorteile der Erfindung werden anhand von Ausführungsbeispielen unter Bezugnahme auf die beigefügten Figuren erläutert. Gleiche und einander entsprechende Teile sind dabei mit übereinstimmenden Bezugszahlen gekennzeichnet. Die darin dargestellten Besonderheiten können einzeln oder in Kombination verwendet werden, um bevorzugte Ausgestaltungen der Erfindung zu schaffen. Es zeigen:
- Fig. 1: ein Ausführungsbeispiel eines Analysehandgeräts,
- Fig. 2: ein Ausführungsbeispiel einer Entnahmeeinrichtung des gezeigten Analysehandgerätes,
- Fig. 3: bis 7 ein weiteres Ausführungsbeispiel einer Entnahmeeinrichtung beim Entnehmen eines Verbrauchsmittels und
- Fig. 8 bis 14: ein weiteres Ausführungsbeispiel eines Analysehandgerätes beim Einführen eines Verbrauchsmittels.

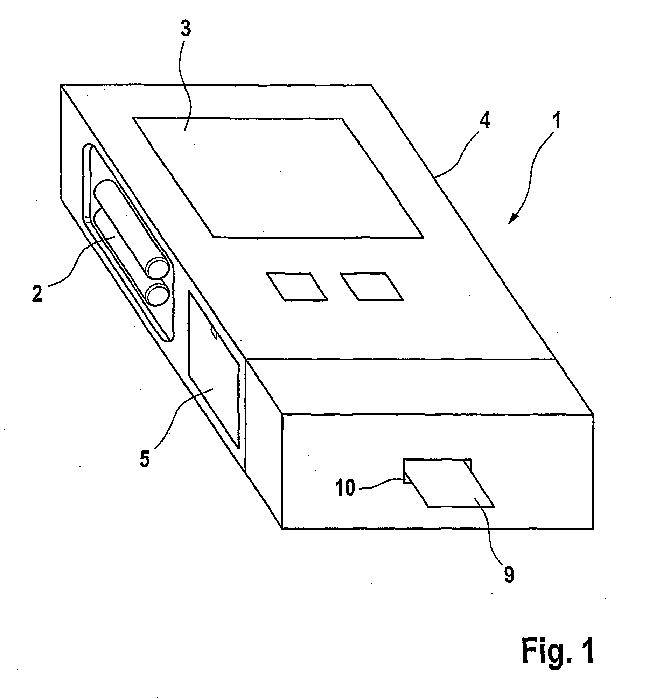

Fig. 1 zeigt ein kompaktes, tragbares Analysehandgerät 1 zum Untersuchen einer Probe, insbesondere einer biologischen Flüssigkeit, auf einen medizinisch bedeutsamen Bestandteil wie beispielsweise Blut, Urin oder Speichel. Das in Fig. 1 gezeigte Analysehandgerät 1 dient zur Bestimmung des Blutglucosegehalts und weist eine Stromquelle 2 in Form handelsüblicher Batterien oder Solarzellen auf. Das Ergebnis einer Untersuchung wird mit einer Anzeigeeinrichtung 3, bevorzugt einem FlüssigkristallDisplay, angezeigt. Das Analysehandgerät 1 weist ein Gehäuse 4 auf, das eine Ladeöffnung 5 zum Aufnehmen eines auswechselbaren Trommelmagazins 6 in ein Magazinfach 7 hat. In dem Magazinfach 7 ist das Trommelmagazin 6 mittels eines Elektromotors 8 schrittweise um seine geometrische Längsachse rotierbar, so daß in dem Trommelmagazin 6 gelagerte analytische Verbrauchsmittel 9 durch eine Gehäuseöffnung 10 des Gehäuses 4 entnommen werden können.

Das im wesentlichen zylindrisch geformte Trommelmagazin 6 hat mehrere ringförmig um seine geometrische Längsachse angeordnete Kammern 11, die analytische Verbrauchsmittel 9 enthalten können. Die Zahl der Kammern 11 kann weitgehend beliebig gewählt werden. In der Regel sind 10 bis 100 Kammern 11 zweckmäßig, bevorzugt sind 15 bis 30 Kammern 11 vorhanden. Jede der Kammern 11 weist an einer Stirnseite des Trommelmagazins 6 eine Entnahmeöffnung 12 zum Entnehmen eines Verbrauchsmittels 9 und eine der Entnahmeöffnung 12 gegenüberliegende Einschuböffnung 13 zum Einführen eines Stößels 14 einer Entnahmeeinrichtung 29 auf. Die Einschuböffnungen 12 und die Entnahmeöffnungen 13 sind zum Schutz der Verbrauchsmittel 9 vor schädlichen Umwelteinflüssen mit einer Siegelfolie verschlossen. Wie in der EP 1 022 565 A2 beschrieben ist, lassen stich mit der als Stößel ausgebildeten Schubstange 14 Verbrauchsmittel 9 zur Verwendung aus den Kammern 11 herausschieben, wobei die Siegelfolie der Einschuböffnung 13 vom Stößel 14 und die Siegelfolie der Entnahmeöffnung 12 von dem Verbrauchsmittel 9 durchstoßen wird.

Bevorzugt sind die Verbrauchsmittel 9 als Teststreifen ausgebildet, auf die eine Probe aufgebracht werden kann. Ein im Teststreifen enthaltenes Reagenz reagiert mit einem medizinisch bedeutsamen Bestandteil der Probe, so daß das Ergebnis der Reaktion mit einem Analysesensor 15 des Analysehandgeräts 1 ausgewertet werden kann. Ein solcher Analysesensor 15 kann beispielsweise ein optischer Sensor sein, der eine Farbänderung eines als Teststreifen ausgebildeten Verbrauchsmittels 9 erfaßt, oder ein elektrischer Sensor sein, der eine Leitfähigkeitsänderung der Probe erfaßt.

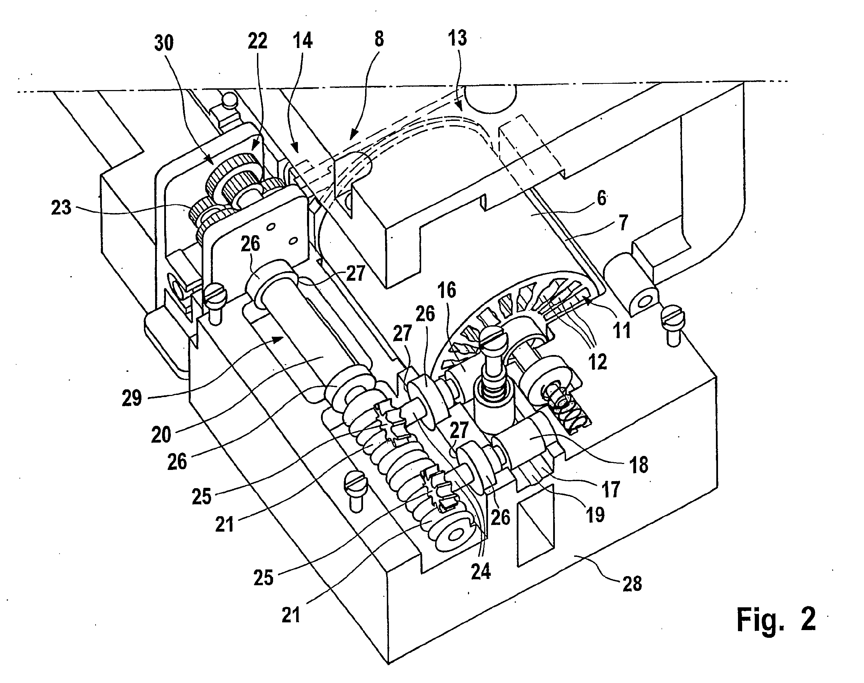

Mittels des Elektromotors 8 läßt sich das Trommelmagazin 6 schrittweise rotieren, so daß nacheinander jede der Entnahmeöffnungen 12 fluchtend mit der Gehäuseöffnung 10 des Gehäuses 4 positioniert und dann mittels des Stößels 14 der Entnahmeeinrichtung 29 ein Verbrauchsmittel 9 aus der zur Entnahme positionierten Kammer 11 herausgeschoben werden kann. Eine Besonderheit des gezeigten Ausführungsbeispiels ist, daß die Entnahmeeinrichtung 29 zusätzlich zu dem Stößel 14 eine in Fig. 2 gezeigte antreibbare Förderwalze 16 umfaßt. Die Förderwalze 16 kann ein aus dem Trommelmagazin 6 herausragendes Verbrauchsmittel 9 greifen und in Entnahmerichtung ganz oder teilweise aus dem Trommelmagazin heraus bewegen. Die Förderwalze 16 ermöglicht es deshalb, den Stößel 14 wesentlich kürzer als bei im Stand der Technik bekannten Geräten auszuführen, da es ausreicht, wenn durch den Stößel 14 das Verbrauchsmittel 9 ein kleines Stück aus der Kammer 11 herausgeschoben werden kann.

Damit ein Verbrauchsmittel 9 nur möglichst wenig aus seiner Kammer 11 herausragen muß, um von der Förderwalze 16 gegriffen werden zu können, ist die Förderwalze 16 bevorzugt zu der Entnahmeöffnung 12 des eingelegten Trommelmagazins 6 unmittelbar benachbart angeordnet. Zwischen der Stirnseite des eingelegten Trommelmagazins 6 und der Förderwalze 16 ist lediglich ein geringer Mindestabstand von etwa 1 mm erforderlich, damit sich die Förderwalze 16 und das eingelegte Trommelmagazin 6 ungestört drehen können. Als weitere Maßnahme, um ein Verbrauchsmittel 9 bereits möglichst nahe an der Entnahmeöffnung 12 mittels der Förderwalze 16 greifen zu können und damit den Stößel 14 möglichst kurz ausbilden zu können, weist die Förderwalze 16 bevorzugt einen kleinen Durchmesser von etwa 3 bis 10 mm, besonders bevorzugt 4 bis 7 mm auf.

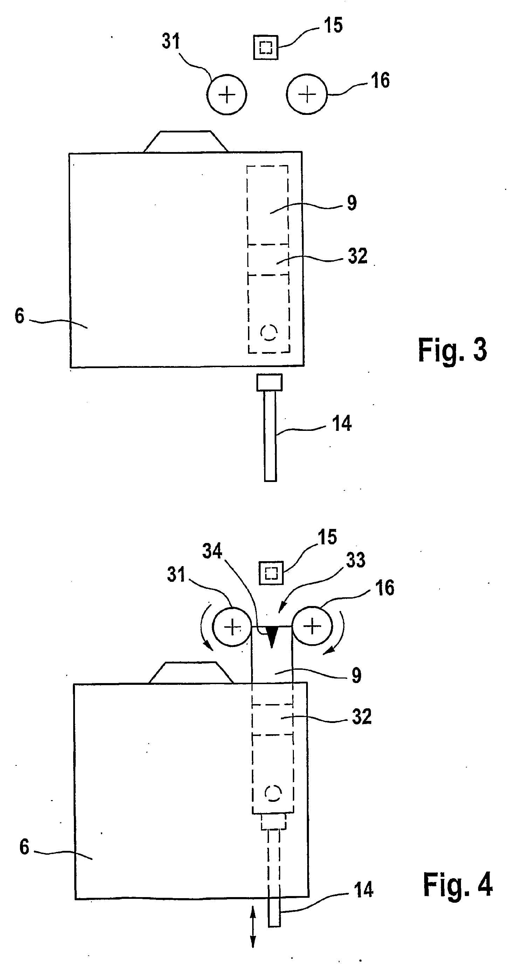

Die Förderwalze 16 bildet zusammen mit einer relativ zu ihr unbeweglichen Förderfläche einen Förderspalt, durch den das Verbrauchsmittel 9 in Förderrichtung bewegt wird. Alternativ kann die Förderwalze 16 - wie in Figur 3 bis 7 gezeigt ist - auch zusammen mit einer ihr gegenüberliegend angeordneten Gegenrolle 31 einen Förderspalt ausbilden. Der Förderspalt weist bevorzugt ein an das Verbrauchsmittel 9 angepaßtes Profil auf, beispielsweise in Form einer Nut in der Förderfläche oder der Gegenrolle, damit ein Testfeld des Verbrauchsmittels 9 beim Entnehmen nicht gequetscht und dadurch beeinträchtigt wird. Die in Fig. 2 gezeigte Ausführungsform, bei der die Förderwalze 16 mit einer unbeweglichen Förderfläche zusammenwirkt, bietet den Vorteil, daß die Entnahmeeinrichtung 29 weniger bewegliche Teile benötigt und deshalb besonders kostengünstig und wenig störanfällig ausgeführt werden kann.

Das Analysehandgerät 1 weist zum Unterstützen eines entnommenen Verbrauchsmittels 9 einen sich in Entnahmerichtung erstreckenden Fördersteg 17 auf. Die zusammen mit der Förderwalze 16 den Förderspalt 33 ausbildende Förderfläche ist Teil des Förderstegs 17, so daß sich dieser von dem Magazinfach 7 bis zur Gehäuseöffnung 10 erstreckt. Auf diese Weise wird ein entnommenes Verbrauchsmittel 9 auf seinem gesamten Förderweg von dem Fördersteg 17 unterstützt und geführt.

Von der Förderwalze 16 in Förderrichtung beabstandet ist eine weitere Förderwalze 18 angeordnet, die zusammen mit dem Fördersteg 17 einen zweiten Förderspalt ausbildet. Während die erste Förderwalze 16 so dicht wie möglich an der Entnahmeöffnung 12 eines eingelegten Trommelmagazins 6 angeordnet ist, damit ein Verbrauchsmittel 9 möglichst wenig aus seiner Kammer 11 herausragen muß, um gegriffen werden zu können, ist die zweite Förderwalze 18 möglichst dicht an der Gehäuseöffnung 10 des Gehäuses 4 angeordnet, damit ein Verbrauchsmittel 9 möglichst weit aus der Gehäuseöffnung 10 des Gehäuses 4 herausgeschoben werden kann. Je weiter nämlich ein beispielsweise als Teststreifen ausgebildetes Verbrauchsmittel 9 aus der Gehäuseöffnung 10 des Gehäuses 4 herausgeschoben werden kann, desto leichter läßt sich auf das Verbrauchsmittel 9 eine Probe, beispielsweise ein Tropfen Blut, aufbringen, ohne daß dabei das Gehäuse 4 von der Probe verschmutzt wird.

Der Fördersteg 17 ist mit einer in Förderrichtung verlaufenden Nut 19 versehen, die vorteilhaft eine zwischen dem Verbrauchsmittel 9 und dem Fördersteg 17 auftretende Reibung minimiert. Während der Fördersteg 17 aus einem möglichst glatten Material mit einem geringen Reibungskoeffizienten, wie beispielsweise Polycarbonat, gefertigt ist, weisen die Förderwalzen 16, 18 bevorzugt eine reibungserhöhend ausgebildete Oberfläche mit einem möglichst großen Reibungskoeffizienten auf. Beispielsweise können die Walzen zur Erhöhung der Reibung eine aufgerauhte Oberfläche aufweisen, aus Hart- oder Weichgummi bestehen oder mit einem gummi-ähnlichen Kunststoff beschichtet sein. Weisen die Verbrauchsmittel 9 eine über ihre Länge variierende Dicke auf, so kann die Förderwalze federgelagert sein, um Dickeunterschieden Rechnung zu tragen.

Bei dem gezeigten Ausführungsbeispiel werden die Förderwalzen 16, 18 und der Stößel 14 der Entnahmeeinrichtung 29 von einem einzigen Antrieb 30 bewegt. Alternativ ist es aber auch möglich, für den Stößel 14 und die Förderwalzen 16, 18 oder sogar für jede der Förderwalzen 16, 18 einen eigenen Mikromotor als Antrieb vorzusehen. Es ist aber kostengünstiger und deshalb bevorzugt, wenn die Entnahmeeinrichtung 29 nur einen Antrieb 30 aufweist, mit dem sowohl die Förderwalzen 16, 18 als auch der Stößel 14 antreibbar sind.

Um eine von dem gemeinsamen Antrieb 30 der Entnahmeeinrichtung 29 erzeugte Bewegung sowohl auf den Stößel 14 als auch die Förderwalzen 16,18 übertragen zu können, weist die Entnahmeeinrichtung 29 eine Gewindestange 20 mit einem Gewinde 21 auf, die sich seitlich neben dem eingelegten Trommelmagazin 6 erstreckt und beidseitig über dessen Stirnseite hinausragt. Bei dem in Fig. 2 gezeigten Ausführungsbeispiel weist die Entnahmeeinrichtung 29 ein Getriebe 22 auf, über das ein zum Antrieb 30 gehörender Elektromotor den Stößel 14 bewegen kann. Die Gewindestange 20 weist ein Zahnrad 23 auf, das mit dem Getriebe 21 zusammenwirkt, so daß die Gewindestange 20 über das Getriebe 22 und das Zahnrad 23 in Rotation versetzbar ist. Das Zahnrad 23 kann dabei als ein separates Bauteil ausgeführt sein, das an der Gewindestange 20 befestigt ist, oder in die Gewindestange 20 integriert sein, indem beispielsweise ein Abschnitt der Gewindestange 20 mit Zähnen versehen ist. Um die Gewindestange 20 so kurz wie möglich ausführen zu können, ist das Zahnrad 23 bevorzugt an oder nahe an einem Ende der Gewindestange 20 und das Gewinde 21 an oder nahe an dem anderen Ende angeordnet.

Eine Rotation der Gewindestange 20 ist über das am anderen Ende der Gewindestange 20 angeordnete Außengewinde 21 auf die Förderwalzen 16, 18 übertragbar. Hierzu ist jede der beiden Förderwalzen 16, 18 mit einer Welle 24 versehen, die ein Zahnrad 25 trägt, das in das Gewinde 21 der Gewindestange 20 eingreift. Die Wellen 24 und die Gewindestange 20 sind mittels Lagerringen 26 gelagert, die mit geringem Reibungswiderstand drehbar in passenden Ausnehmungen 27 eines Trägers 28 liegen.

Bevorzugt sind die Förderwalzen 16, 18 um ihre geometrischen Längsachsen sowohl im Uhrzeigersinn als auch gegen den Uhrzeigersinn antreibbar, um ein Verbrauchsmittel 9 sowohl in Entnahmerichtung als auch in entgegengesetzter Richtung bewegen zu können. Diese Maßnahme ermöglicht es, ein Verbrauchsmittel 9, beispielsweise einen Teststreifen, möglichst weit aus der Gehäuseöffnung 10 des Gehäuses 4 herauszuschieben, um das Aufbringen einer Probe zu erleichtern und anschließend das Verbrauchsmittel 9 wieder in das Analysehandgerät 1 hineinzuziehen. Dadurch ist es möglich, den Analysesensor 15 an einer geschützten Stelle im Innern des Gehäuses 4 anzuordnen, wo störende Umwelteinflüsse, wie beispielsweise Streulicht, minimiert sind. Bevorzugt ist der Analysesensor 15 zwischen den beiden Förderwalzen 16, 18, insbesondere auf der den Förderwalzen 16, 18 gegenüberliegenden Seite des Förderstegs 17 angeordnet. Der Fördersteg 17 ist zwischen den beiden Förderwalzen 16, 18 mit einer Ausnehmung versehen, so daß der Analysesensor 15 eine auf einem Verbrauchsmittel 9 aufgebrachte Probe erfassen kann. Die in Förderrichtung verlaufende Nut 19 des Förderstegs 17 ist dabei in ihrer Breite und Tiefe so bemessen, daß eine auf das Verbrauchsmittel 9 aufgebrachte Probe nicht mit dem Fördersteg 17 in Kontakt kommt.

Sind die Förderwalzen 16, 18 um ihre geometrische Längsachse sowohl im Uhrzeigersinn als auch gegen den Uhrzeigersinn drehbar, so daß das Verbrauchsmittel 9 sowohl in Entnahmerichtung als auch in entgegengesetzte Richtung bewegt werden kann, so ist es vorteilhaft möglich, ein benutztes Verbrauchsmittel 9 nach abgeschlossener Untersuchung wieder in seine Kammer 11 des Trommelmagazins 6 einzubringen. Um das Remagazinieren eines benutzten Verbrauchsmittels 9 zu unterstützen, ist der Stößel 14 mit einem Greifelement ausgestattet, das mit einem Verbrauchsmittel 9 in Wirkeingriff treten kann und es ermöglicht, über den Stößel 14 auch Zugkräfte auf ein Verbrauchsmittel 9 ausüben zu können. Beispielsweise kann das Greifelement als ein Elektromagnet, der ein Eisenteil des Verbrauchsmittels anzieht, oder als ein mechanischer Haken ausgebildet sein, der bei einer vorgegebenen Zugkraft, wie sie von der Förderwalze 16 ausgeübt wird, umklappt und das Verbrauchsmittel 9 freigibt.

Vorteilhaft können auf diese Weise alle in einem Trommelmagazin 6 enthaltenen Verbrauchsmittel 9 auf einmal entsorgt werden und ein Benutzer braucht nicht mehr nach jeder Untersuchung das benutzte Verbrauchsmittel 9 einzeln zu entsorgen.

Die Figuren 3 bis 7 zeigen in einer schematischen Darstellung ein weiteres Ausführungsbeispiel einer Entnahmevorrichtung beim Entnehmen eines Verbrauchsmittels 9 aus einem Trommelmagazin 6. Im Unterschied zu dem vorhergehend beschriebenen Ausführungsbeispiel wird bei der in den Figuren 3 bis 7 gezeigte Entnahmeeinrichtung 29 der Förderspalt 33 nicht durch die Förderwalze 16 und eine relativ zu ihr unbewegliche Förderfläche, sondern durch die Förderwalze 16 und eine ihr gegenüberliegend angeordnete Gegenrolle 31 ausgebildet. Bei dem in den Figuren 3 bis 7 gezeigten Ausführungsbeispiel können sowohl die Förderwalze 16 als auch die Gegenrolle 31 jeweils antreibbar ausgebildet sein. Es genügt jedoch, wenn lediglich die Förderwalze 16 antreibbar ist. Für die Gegenrolle 31 reicht es aus, wenn diese drehbar gelagert ist, so daß sie durch ein durch den Förderspalt 33 hindurch tretendes Verbrauchsmittel 9 in Rotation versetzbar ist.

Bei dem in den Figuren 3 bis 7 gezeigten Ausführungsbeispiel sind die Verbrauchsmittel 9 als Teststreifen ausgebildet, die ein Testfeld 32 zum Aufnehmen einer Probe aufweisen. Damit das Testfeld 32 beim Entnehmen des Verbrauchsmittels 9 aus dem Trommelmagazin 6 nicht beeinträchtigt wird und eine darauf befindliche Probe nicht die Entnahmeeinrichtung 29, insbesondere nicht die Förderwalze 16 und die Gegenrolle 31, verschmutzen kann, tritt bei dem gezeigten Ausführungsbeispiel das Verbrauchsmittel 9 so durch den Förderspalt, daß sich das Testfeld 32 des Verbrauchsmittels 9 quer zur geometrischen Rotationsachse der Förderwalze 16 erstreckt. Auf diese Weise wird die Gefahr einer Beeinträchtigung des Testfeldes 32 des Verbrauchsmittels 9 noch weiter reduziert, als sich dies bei dem anhand von Figur 2 beschriebenen Ausführungsbeispiel durch einen Förderspalt 33 mit einem an das Verbrauchsmittel 9 angepaßten Profil, beispielsweise in Form einer Nut 19 in dem die Förderfläche ausbildenden Fördersteg 17, erreichen läßt.

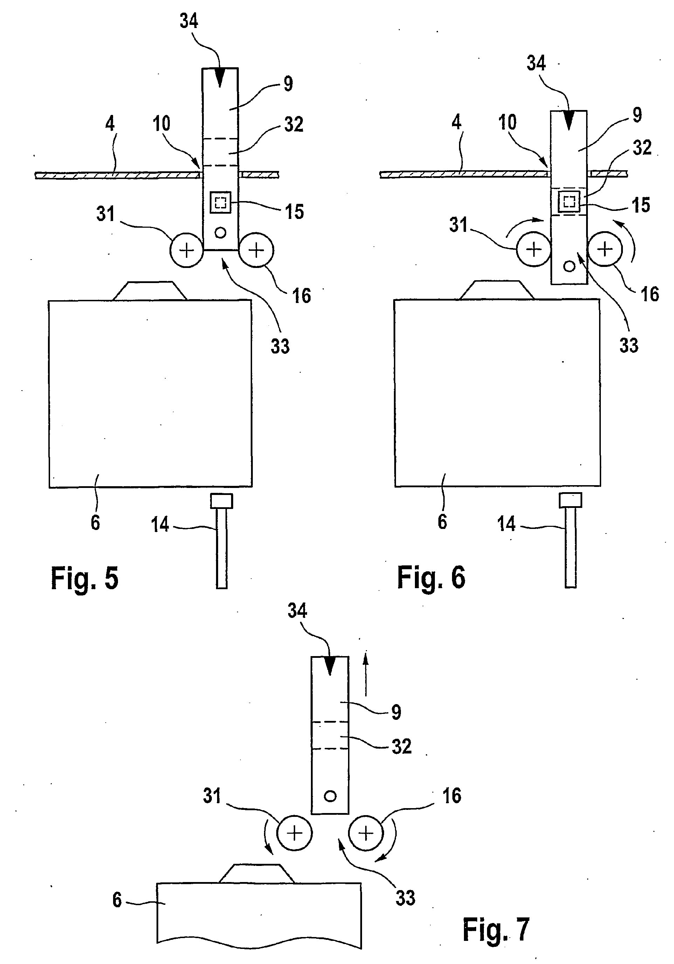

Figur 3 zeigt das Trommelmagazin 6 mit einem darin enthaltenen Verbrauchsmittel 9 zusammen mit der Entnahmeeinrichtung 29 in der Ausgangslage. Wie in Figur 4 zu sehen ist, wird das Verbrauchsmittel 9 mit dem Stößel 14 aus dem Trommelmagazin 6 herausgeschoben und von der Förderwalze 16 gegriffen, sobald es in den Förderspalt 33 zwischen der Förderwalze 16 und der Gegenrolle 31 hineinragt. Figur 5 zeigt wie das Verbrauchsmittel 9 von der Förderwalze 16 aus der Gehäuseöffnung 10 des Analysehandgeräts 1 herausgeschoben wird, so daß eine Probe, beispielsweise ein Tropfen Blut, auf eine Probenauftragfläche 34 aufgebracht werden kann, von der aus sie zum Testfeld 32 gelangt. Anschließend wird, wie in Figur 6 gezeigt, das Verbrauchsmittel 9 von der sich nun in entgegengesetzter Richtung drehenden Förderwalze 16 wieder in das Analysehandgerät 1 hineingezogen, so daß das Testfeld 32 für die Untersuchung der Probe vor dem Analysesensor 15 positioniert ist. Nachdem der Stößel 14 das Verbrauchsmittel 9 aus dem Trommelmagazin 6 herausgeschoben hat, kehrt er in seine in Figur 6 gezeigte Ausgangsposition zurück. Nach abgeschlossener Untersuchung wird das Verbrauchsmittel 9, wie in Figur 7 gezeigt, von der Förderwalze 16 wieder in Entnahmerichtung bewegt und aus dem Analysehandgerät 1 ausgeworfen.

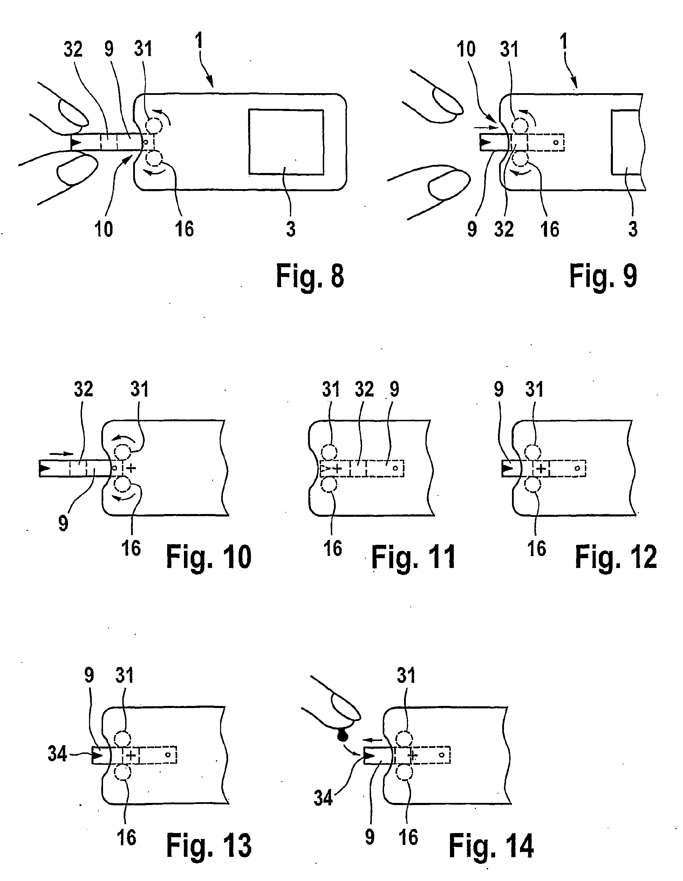

Die Figuren 8 bis 14 zeigen ein weiteres Ausführungsbeispiel eines Analysehandgerätes 1 beim Einführen eines Verbrauchsmittels 9. Dieses Analysehandgerät 1 unterscheidet sich von dem im vorhergehenden beschriebenen Analysehandgerät 1 im wesentlichen dadurch, daß es keine Ladeöffnung zur Aufnahme eines auswechselbaren Trommelmagazins aufweist. Bei dem in Figuren 8 bis 14 gezeigten Analysehandgerät 1 werden analytische Verbrauchsmittel 9 in Form von Teststreifen deshalb dem Gerät extern durch die Gehäuseöffnung 10 zugeführt. Beim Einführen eines Verbrauchsmittels 9 in die Gehäuseöffnung 10 wird ein Mikroschalter (nicht gezeigt) betätigt, durch den ein Antrieb der Förderwalze 16 und der Gegenrolle 31 eingeschaltet wird. Wie in Fig. 9 gezeigt ist, wird das Verbrauchsmittel 9 daraufhin von Förderwalze 16 und der Gegenrolle 31 gegriffen und entlang des Förderwegs in das Geräteinnere hineingezogen.

Werden die analytischen Verbrauchsmittel 9, wie bei dem gezeigten Ausführungsbeispiel, nicht einem in dem Analysehandgerät 1 gelagerten Trommelmagazin 6 entnommen, sondern extern zugeführt, so besteht eine erhöhte Gefahr, daß die Verbrauchsmittel 9 durch Alterung oder schädliche Umwelteinflüsse, wie beispielsweise Licht, Feuchtigkeit oder Staub, verdorben sind. Ein Verbrauchsmittel 9 wird deshalb aus der in Fig. 10 gezeigten Eingabeposition zunächst in die in Fig. 11 gezeigte Testposition befördert, in der es darauf überprüft wird, ob es noch nicht verdorben ist. Dies geschieht mittels einer optischen Meßeinrichtung, mit der ein Folienweißwert des Verbrauchsmittels 9 bestimmt wird. Ein als Teststreifen ausgeführtes Verbrauchsmittel 9 weist zu diesem Zweck, beispielsweise im Bereich des Testfeldes 32, einen Abschnitt mit einer weißen Kunststoffolie auf, die sich mit zunehmender Alterung und Feuchtigkeitsaufnahme verfärbt. Durch eine Bestimmung des Folienweißwertes kann festgestellt werden, ob das eingelegte Verbrauchsmittel 9 verdorben ist. Ist ein Verbrauchsmittel 9 verdorben, so wird es mittels der Förderwalze 16 und der Gegenrolle 31 aus dem Analysehandgerät 1 ausgeworfen. Ist das Verbrauchsmittel 9 noch funktionsfähig, so wird es mittels der Förderwalze 16 und der Gegenrolle 31 entlang des Förderweges in die in Fig. 14 gezeigte Probenaufgabeposition gebracht. In der Probenauftragposition ragt das Verbrauchsmittel 9 mit einem Ende aus der Gehäuseöffnung 10 heraus, so daß eine Probe, beispielsweise ein Blutstropfen auf die Probenauftragfläche 34 aufgebracht werden kann.

Ein wichtiger Vorteil des beschriebenen Anlaysehandgeräts 1 ist, daß das Verbrauchsmittel 9 in der in Fig. 14 gezeigten Probenauftragposition soweit aus der Gehäuseöffnung 10 herausragen kann, daß sich eine Probe leicht auf die Probenauftragfläche 34 aufbringen läßt, ohne das Analysehandgerät 1 damit zu verschmutzen. Durch ein in der Probenauftragposition relativ weit aus der Gehäuseöffnung 10 herausragendes Verbrauchsmittel 9 wird für einen Benutzer auch leichter deutlich gemacht, daß nun eine Probe auf die Probenauftragfläche 34 aufgebracht werden soll. Insbesondere läßt sich das Testfeld 32, dem die Probe beispielsweise durch Kapillarkräfte zugeführt wird, so nahe an der Probenauftragfläche 34 anordnen, daß das benötigte Probenvolumen minimal ist.

### Bezugszeichenliste

- 1: Analysehandgerät
- 2: Stromquelle
- 3: Anzeigeeinrichtung
- 4: Gehäuse
- 5: Ladeöffnung
- 6: Trommelmagazin
- 7: Magazinfach
- 8: Elektromotor
- 9: Verbrauchsmittel
- 10: Gehäuseöffnung
- 11: Kammer
- 12: Entnahmeöffnung
- 13: Einschuböffnung
- 14: Schubstange
- 15: Analysesensor
- 16: Förderwalze
- 17: Fördersteg
- 18: Förderwalze
- 19: Nut
- 20: Gewindestange
- 21: Gewinde
- 22: Getriebe
- 23: Zahnrad
- 24: Welle
- 25: Zahnrad
- 26: Lagerring
- 27: Ausnehmung
- 28: Träger
- 29: Entnahmeeinrichtung
- 30: Antrieb
- 31: Gegenrolle
- 32: Testfeld
- 33: Förderspalt
- 34: Probenauftragfläche

## Patentansprüche

1. Analysehandgerät zum Untersuchen einer Probe, insbesondere einer biologischen Flüssigkeit, auf einen medizinisch bedeutsamen Bestandteil, umfassend
einen Analysesensor (15), dem auf einem Förderweg ein analytisches Verbrauchsmittel zuführbar ist,
eine Anzeigeeinrichtung (3),
ein Gehäuse (4), das eine Gehäuseöffnung (10) für ein analytisches Verbrauchsmittel (9) aufweist,
an die der Förderweg anschließt,
und eine antreibbare Förderwalze (16,18), mit der ein in den Förderweg ragendes Verbrauchsmittel (9) gegriffen und entlang des Förderwegs bewegt werden kann,
**dadurch gekennzeichnet, dass**
die Förderwalze (16,18) um ihre geometrische Längsachse sowohl im Uhrzeigersinn als auch gegen den Uhrzeigersinn antreibbar ist, um ein Verbrauchsmittel (9) sowohl in Entnahmerichtung als auch in entgegengesetzter Richtung bewegen zu können.

2. Analysehandgerät nach Anspruch 1, **dadurch gekennzeichnet, daß** das Gehäuse (4) eine Ladeöffnung (5) zum Aufnehmen eines auswechselbaren Trommelmagazins (6) aufweist, das analytische Verbrauchsmittel (9), insbesondere Teststreifen, enthalten kann und an einer Stirnseite mindestens eine Entnahmeöffnung (12) aufweist, und
daß das Gehäuse (4) eine Entnahmeeinrichtung (29) zum Entnehmen eines der analytischen Verbrauchsmittel (9) aus dem Trommelmagazin (6) umgibt, wobei
die Entnahmeeinrichtung (29) die antreibbare Förderwalze (16,18) umfaßt, die ein aus dem Trommelmagazin (6) herausragendes und in den Förderweg hineinragendes Verbrauchsmittel (9) greifen und in einer Entnahmerichtung ganz oder teilweise aus dem Trommelmagazin (6) heraus bewegen kann.

3. Analysehandgerät nach Anspruch 1 oder 2, **dadurch gekennzeichnet, daß** es eine Schubstange (14) aufweist, mittels der ein Verbrauchsmittel (9) so weit aus einer Kammer herausgeschoben werden kann, daß es von der Förderwalze (16,18) gegriffen und in Entnahmerichtung bewegt werden kann.

4. Analysehandgerät nach einem der vorhergehenden Ansprüche, **dadurch gekennzeichnet, daß** die Förderwalze (16,18) zusammen mit einer Gegenrolle (31) einen Förderspalt (33) bildet, durch den das Verbrauchsmittel (9) hindurch bewegt wird.

5. Analysehandgerät nach einem der Ansprüche 1 bis 3, **dadurch gekennzeichnet, daß** die Förderwalze (16,18) zusammen mit einer relativ zu ihr unbeweglichen Förderfläche einen Förderspalt (33) ausbildet, durch den das Verbrauchsmittel (9) bewegt wird.

6. Analysehandgerät nach Anspruch 3 oder 4, **dadurch gekennzeichnet, daß** der Förderspalt (33) ein an das Verbrauchsmittel (9) angepaßtes Profil aufweist.

7. Analysehandgerät nach Anspruch 6, **dadurch gekennzeichnet, daß** die Förderfläche oder die Gegenrolle eine in Förderrichtung verlaufende Nut (19) aufweisen.

8. Analysehandgerät nach einem der vorhergehenden Ansprüche, **dadurch gekennzeichnet, daß** es zum Unterstützen eines entnommenen Verbrauchsmittels (9) einen sich entlang des Förderwegs erstreckenden Fördersteg (17) aufweist.

9. Analysehandgerät nach Anspruch 8, **dadurch gekennzeichnet, daß** die Förderfläche Teil des Förderstegs (17) ist.

10. Analysehandgerät nach einem der Ansprüche 2 bis 9, **dadurch gekennzeichnet, daß** die Förderwalze (16,18) unmittelbar benachbart zu der die Entnahmeöffnung (12) aufweisenden Stirnseite eines eingelegten Trommelmagazins (6) angeordnet ist.

11. Analysehandgerät nach einem der vorhergehenden Ansprüche, **dadurch gekennzeichnet, daß** die Förderwalze (16,18) eine reibungserhöhend ausgebildete Oberfläche aufweist.

12. Analysehandgerät nach einem der Ansprüche 2 bis 11, **dadurch gekennzeichnet, daß** die Entnahmeeinrichtung (29) eine weitere Förderwalze (18) zum Bewegen eines Verbrauchsmittels (9) aufweist, wobei die erste Förderwalze (16) und die weitere Förderwalze (18) entlang des Förderwegs voneinander beabstandet angeordnet sind.

13. Analysehandgerät nach einem der Ansprüche 2 bis 12, **dadurch gekennzeichnet, daß** die Entnahmeeinrichtung (29) eine Schubstange (14) aufweist, die zum Herausschieben eines Verbrauchsmittels (9) aus dem Trommelmagazin (6) in eine der Entnahmeöffnung (12) gegenüberliegende Einschuböffnung (13) einführbar ist.

14. Analysehandgerät nach Anspruch 13, **dadurch gekennzeichnet, daß** die Entnahmeeinrichtung (29) einen Antrieb (30) aufweist, durch den sowohl die Förderwalze (16,18) als auch die Schubstange (14) gemeinsam antreibbar sind.

15. Analysehandgerät nach Anspruch 14, **dadurch gekennzeichnet, daß** die Entnahmeeinrichtung (29) eine Gewindestange (20) mit einem Gewinde (21) aufweist, die sich seitlich neben einem eingelegten Trommelmagazin (6) erstreckt und mit einer Welle (24) zum Antreiben der Förderwalze (16,18) zusammenwirkt.

16. Analysehandgerät nach Anspruch 15, **dadurch gekennzeichnet, daß** der Antrieb (30) ein Getriebe (22) zum Bewegen des Stößels (14) aufweist, das über ein an der Gewindestange (20) angebrachtes Zahnrad (23) mit diesem zusammenwirkt.

17. Analysehandgerät nach einem der Ansprüche 2 bis 16, **dadurch gekennzeichnet, daß** die Entnahmeeinrichtung (29) derart ausgebildet ist, daß ein benutztes Verbrauchsmittel (9) wieder in eine Kammer (11) des Trommelmagazins (6) einbringbar ist.

## Claims

1. A handheld analysis device for analyzing a sample, in particular a biological liquid, for a medically significant component, comprising
an analysis sensor (15), to which an analytic consumable may be supplied along a conveyance path,
a display unit (3),
a housing (4), which has a housing opening (10) for an analytic consumable (9),
the conveyance path following on the housing opening (10),
and a drivable conveyance roll (16, 18), by which a consumable (9) protruding into the conveyance path may be gripped and moved along the conveyance path,
**characterized in that**
the conveyance roll (16, 18) is drivable around its geometrical longitudinal axis both clockwise and also counterclockwise in order to be able to move a consumable (9) both in the removal direction and also in the opposite direction.

2. The handheld analysis device according to claim 1, **characterized in that** the housing (4) has a loading opening (5) for receiving a replaceable drum magazine (6), which may contain analytic consumables (9), particularly test strips, and has at least one removal opening (12) on a front face, and
the housing (4) encloses a removal facility (29) for removing one of the analytic consumables (9) from the drum magazine (6), wherein
the removal facility (29) comprises the drivable conveyor roll (16, 18), which may grip a consumable (9) projecting out of the drum magazine (6) and into the conveyance path and move it entirely or partially out of the drum magazine (6) in a removal direction.

3. The handheld analysis device according to claim 1 or 2, **characterized in that** it comprises a pushrod (14), by which a consumable (9) can be pushed so far out of a chamber that it may be gripped by the conveyance roll (16, 18) and moved in the removal direction.

4. The handheld analysis device according to any one of the preceding claims, **characterized in that** the conveyance roll (16, 18), together with a counter roll (31), forms a conveyance gap (33), through which the consumable (9) is moved.

5. The handheld analysis device according to any one of claims 1 to 3, **characterized in that** the conveyance roll (16, 18) and a conveyance surface stationary in relation thereto form together a conveyance gap (33), through which the consumable (9) is moved.

6. The handheld analysis device according to claim 3 or 4, **characterized in that** the conveyance gap (33) has a profile tailored to the consumable (9).

7. The handheld analysis device according to claim 6, **characterized in that** the conveyance surface or the counter roll has a groove (19) running in the conveyance direction.

8. The handheld analysis device according to any one of the preceding claims, **characterized in that** it has a conveyance base (17) extending along the conveyance path to support a removed consumable (9).

9. The handheld analysis device according to claim 8, **characterized in that** the conveyance surface is part of the conveyance base (17).

10. The handheld analysis device according to any one of claims 2 to 9, **characterized in that** the conveyance roll (16, 18) is situated directly adjacent to the front face, having the removal opening (12), of an inserted drum magazine (6).

11. The handheld device according to any one of the preceding claims, **characterized in that** the conveyance roll (16, 18) has a surface adapted to increase friction.

12. The handheld analysis device according to any one of claims 2 to 11, **characterized in that** the removal facility (29) has a further conveyance roll (18) for moving a consumable (9), the first conveyance roll (16) and the further conveyance roll (18) being situated at a distance from one another along the conveyance path.

13. The handheld analysis device according to any one of claims 2 to 12, **characterized in that** the removal facility (29) has a pushrod (14), which may be inserted into an insertion opening (13) diametrically opposite the removal opening (12) to push a consumable (9) out of the drum magazine (6).

14. The handheld analysis device according to claim 13, **characterized in that** the removal facility (29) has a drive (30), by which both the conveyance roll (16, 18) and also the pushrod (14) are jointly drivable.

15. The handheld analysis device according to claim 14, **characterized in that** the removal facility (29) has a threaded rod (20) having a thread (21), which extends laterally next to an inserted drum magazine (6) and works together with a shaft (24) to drive the conveyance roll (16, 18).

16. The handheld analysis device according to claim 15, **characterized in that** the drive (30) has a transmission (22) for moving the pushrod (14), which works together with the threaded rod (20) via a gearwheel (23) attached thereto.

17. The handheld analysis device according to any one of claims 2 to 16, **characterized in that** the removal facility (29) is implemented in such a manner that a used consumable (9) may be reintroduced into a chamber (11) of the drum magazine (6).

## Revendications

1. Appareil d'analyse manuel pour l'analyse d'un composant médicalement pertinent dans un échantillon, en particulier d'un liquide biologique, comprenant :
un capteur d'analyse (15) alimentable par un consommable analytique sur un trajet de transport,
un dispositif afficheur (3),
un boîtier (4) présentant une ouverture de boîtier (10) pour un consommable analytique (9),
à laquelle le trajet de transport est raccordé,
et un rouleau de transport (16, 18) entraînable, au moyen duquel un consommable analytique (9) pénétrant dans le trajet de transport peut être saisi et déplacé sur le trajet de transport,
**caractérisé en ce que** le rouleau de transport (16, 18) est entraînable autour de son axe géométrique longitudinal tant dans le sens des aiguilles d'une montre que dans le sens anti-horaire, pour pouvoir déplacer un consommable analytique (9) tant dans la direction de prélèvement que dans la direction opposée.

2. Appareil d'analyse manuel selon la revendication 1, **caractérisé en ce que** le boîtier (4) présente une ouverture de chargement (5) pour le logement d'un magasin à tambour (6) renouvelable pouvant contenir des consommables analytiques (9), en particulier des bandes de test, et présente au moins une ouverture de prélèvement (12) sur une face frontale, et
**en ce que** le boîtier (4) entoure un dispositif de prélèvement (29) pour le prélèvement d'un des consommables analytiques (9) hors du magasin à tambour (6),
le dispositif de prélèvement (29) comprenant le rouleau de transport (16, 18) entraînable, lequel peut saisir un consommable analytique (9) sortant du magasin à tambour (6) et pénétrant dans le trajet de transport, et déplacer celui-ci dans une direction de prélèvement en l'extrayant totalement ou partiellement du magasin à tambour (6).

3. Appareil d'analyse manuel selon la revendication 1 ou 2, **caractérisé en ce qu'**il comporte une tige de poussée (14) au moyen de laquelle un consommable (9) peut être ressorti d'un compartiment sur une longueur lui permettant d'être saisi par le rouleau de transport (16, 18) et déplacé dans la direction de déplacement.

4. Appareil d'analyse manuel selon l'une des revendications précédentes, **caractérisé en ce que** le rouleau de transport (16, 18) forme une fente de transport (33) avec un contre-rouleau (31), au travers de laquelle le consommable (9) est déplacé.

5. Appareil d'analyse manuel selon l'une des revendications 1 à 3, **caractérisé en ce que** le rouleau de transport (16, 18) forme une fente de transport (33) avec une surface de transport immobile par rapport à lui, par laquelle le consommable (9) est déplacé.

6. Appareil d'analyse manuel selon la revendication 3 ou 4, **caractérisé en ce que** la fente de transport (33) présente un profil adapté au consommable (9).

7. Appareil d'analyse manuel selon la revendication 6, **caractérisé en ce que** la surface de transport ou le contre-rouleau présentent une rainure (19) s'étendant dans la direction de transport.

8. Appareil d'analyse manuel selon l'une des revendications précédentes, **caractérisé en ce qu'**il présente un méplat de transport (17) s'étendant le long du trajet de transport, pour le support d'un consommable (9) prélevé.

9. Appareil d'analyse manuel selon la revendication 8, **caractérisé en ce que** la surface de transport fait partie du méplat de transport (17).

10. Appareil d'analyse manuel selon l'une des revendications 2 à 9, **caractérisé en ce que** le rouleau de transport (16, 18) est directement contigu à la face frontale d'un magasin à tambour (6) inséré, laquelle présente l'ouverture de prélèvement (12).

11. Appareil d'analyse manuel selon l'une des revendications précédentes, **caractérisé en ce que** le rouleau de transport (16, 18) présente une surface formée pour accroître la friction.

12. Appareil d'analyse manuel selon l'une des revendications 2 à 11, **caractérisé en ce que** le dispositif de prélèvement (29) comporte un autre rouleau de transport (18) pour le déplacement d'un consommable (9), le premier rouleau de transport (16) et l'autre rouleau de transport (18) étant espacés entre eux le long du trajet de transport.

13. Appareil d'analyse manuel selon l'une des revendications 2 à 12, **caractérisé en ce que** le dispositif de prélèvement (29) comporte une tige de poussée (14) insérable dans une ouverture de poussée (13) opposée à l'ouverture de prélèvement (12) pour pousser un consommable (9) hors du magasin à tambour (6).

14. Appareil d'analyse manuel selon la revendication 13, **caractérisé en ce que** le dispositif de prélèvement (29) comporte un entraînement (30) au moyen duquel le rouleau de transport (16, 18) ainsi que la tige de poussée (14) sont entraînables ensemble.

15. Appareil d'analyse manuel selon la revendication 14, **caractérisé en ce que** le dispositif de prélèvement (29) comporte une tige filetée (20) avec un filet (21), laquelle s'étend latéralement à côté d'un magasin à tambour (6) inséré et coopère avec un arbre (24) pour entraîner le rouleau de transport (16, 18).

16. Appareil d'analyse manuel selon la revendication 15, **caractérisé en ce que** l'entraînement (30) comporte un engrenage (22) pour le déplacement du poussoir (14), lequel coopère avec ce dernier au moyen d'un pignon (23) disposé sur la tige filetée (20).

17. Appareil d'analyse manuel selon l'une des revendications 2 à 16, **caractérisé en ce que** le dispositif de prélèvement (29) est réalisé de manière à permettre la réintroduction d'un consommable (9) utilisé dans un compartiment (11) du magasin à tambour (6).
